# EUROPEAN PATENT APPLICATION

(11) **EP 4 071 472 A1**
(43) Date of publication of application: **12.10.2022**
(21) Application number: 20895200.2
(22) Date of filing: 25.11.2020
(51) Int. Cl.: G01N 33/50, G01N 33/02

(54) **OPTICAL METHOD FOR DETECTING A TARGET MOLECULE BY MEANS OF THE AMPLIFICATION IN THE INTERFERENCE RESPONSE, RESULTING FROM THE REFRACTIVE INDEX AND DISPERSION**

(30) Priority: 02.12.2019 ES 201931066
(71) Applicant: Universidad Politécnica De Madrid, 28040 Madrid (ES); Bio Optical Detection, S.L., 28223 Mejorada del Campo (Madrid) (ES)
(72) Inventor: HOLGADO BOLAÑOS, Miguel, 28040 Madrid (ES); DÍAZ PERALES, Araceli, 28040 Madrid (ES); GARRIDO ARANDIA, María, 28040 Madrid (ES); LÓPEZ ESPINOSA, Rocío, 28040 Madrid (ES); ROMERO SAHAGÚN, Alejandro, 28040 Madrid (ES); LAGUNA HERAS, María Fe, 28040 Madrid (ES); FERNÁNDEZ PACIOS, Luis, 28040 Madrid (ES); SANTAMARÍA FERNÁNDEZ, Beatriz, 28040 Madrid (ES); RAMÍREZ ALONSO, Yolanda, 28223 Mejorada del Campo (Madrid) (ES); SANZA GUTIÉRREZ, Francisco Javier, 28223 Mejorada del Campo (Madrid) (ES)
(74) Representative: Elzaburu S.L.P.
(86) International application number: PCT/ES2020/070735
(87) International publication number: WO 2021/111020

(57) **Abstract**

The invention relates to an optical method for detecting at least one target molecule (TM) contained in a sample at a determined concentration, which comprises: (a) bringing a sample containing the TM into contact, in a liquid medium, with a solution containing nanoparticles (NPs), the surface of the NPs having been coated or functionalised with at least one type of specific bioreceptor (BR) of the target molecule to be detected (NP-BR), such that the BRs specifically recognise the TM, thus forming conjugates of the NP-BRs with the TMs (NP-BR-TMs); (b) separating the nanoparticles conjugates (NP-BR-TMs and/or NP-BRs) formed in the previous

## Description

### TECHNICAL FIELD

The present invention belongs to the technical sector of immunological analysis and/or diagnosis using optical biosensors which work by optical interference of light, such as those described in the invention *Optica! detection system for labelling-free high-sensitivity bioassays* (ES2574138 (T3) - 2016- 06-15) or in the scietific review publication: Emerging applications of label-free optical biosensors (G. Zanchetta, et al. Nanophotonics, 6, 1-18 (2017). More specifically, the invention described herein relates to a method for the detection and/or quantification of a target molecule (TM), in particular an immunologically reactive molecule or one with bioreceptor-antigen affinity, wherein the method comprises enriching the target molecule (also referred to as target analyte) in a sample by using functionalized nanoparticles, and subsequently analyzing it by direct optical reading of the reflection and/or transmission of the system, the interference response of which will be affected by the conjugates of the nanoparticles specifically recognized on its surface due to variation of the real part of the refractive index and possible loss of intensity due to variation of the complex part of the refractive index and/or scattering of the aforementioned complexes in the reflected or directly transmitted light, without the need to use additional detection methods.

Additionally, the present invention relates to a method that can be applied to multiple optical reading systems or devices, preferably portable, such as that described in the invention *INTERFEROMETRIC DETECTION METHOD* (EP2880396 (A1) - 2015-06-10) and subsequently reported for example in the scientific publications: Towards reliable optical label-free point-of-care (PoC) biosensing devices (Sensors and Actuators B 236 (2016) 765-772) or Description of an Advantageous Optical Label-Free Biosensing Interferometric Read-Out Method to Measure Biological Species (Sensors 2014, 14, 3675-3689), which allows the diagnosis of diseases with an immunological basis in a reasonable time.

### BACKGROUND ART

So far the most common high-sensitivity *in-vitro* detection systems available on the market for biomolecular analysis are based on chemical development and amplification, with the ELISA technique (Enzyme-Linked Immunosorbent Assay) being the most common and established on the market. There exist a wide variety of devices; to refer to a specific case, notable in the allergy sector are IMMUNOCAP, IMMUNOCAP ISAC, which can offer quantitative information and particularly a qualitative measurement (yes/no response) with respect to the presence of specific IgE allergy antibodies, which does not allow to define the sensitization profile resolved by components for each patient, necessary for complex cases. In addition, these devices offer only a limited range of food allergens that can be tested, using raw allergen extracts instead of individual allergens, which can lead to misleading information. Additionally, this type of devices require highly qualified facilities and personnel.

The number of bibliographic references relating to the field of optical biosensors is very high, both in the scientific literature and in patent documents. In many of these documents, the physical principle of detection of optical biosensors is based on light interference phenomena, by the change in the refractive index when biological material accumulates on a sensor surface. However, these devices have not yet been marketed in mass and, therefore, traditional methods based on chemical amplification or development remain the usual and commercially available ones, including both nitrocellulose strips based on lateral flow that work by colorimetry, as well as the aforementioned ELISA. One of the possible causes may be the requirement of the high sensitivity required for the different applications of these sensors, as well as the difficulty in working with real biological samples with a complex matrix. Among other factors, the concentration of the biomolecules to be recognized is generally very small, the complexity when working with real samples is high due to the high number of other agents (biological or not) present that can generate a high non-specific adsorption and to the lack of specificity. In addition, this problem is amplified and there is a limitation in the ability to detect molecules of very low molecular mass, whose impact on the increase in the refractive index on the sensor surface is much smaller, such that the sensitivity factor of the transducer must be much greater, while at the same time the requirements of error or measurement uncertainty in the reading of the device are more critical to reach the necessary detection limit, that can be estimated as the ratio between the measurement uncertainty of the reading and the sensitivity of the transducer. In short, these requirements mean that, in order to reach a detection limit in accordance with the needs, the system must be very competitive, the sensitivity of the transducer must be very high, the reading uncertainty very small and, additionally, the system must be able to specifically recognize the target biomolecules, including those of very low molecular mass, in real samples with a multitude of components or agents in a highly specific way, avoiding as far as possible any non-specific detection that could invalidate the analysis.

In summary, these very strict requirements of specificity, sensitivity and detection limit pose a great difficulty when developing a method and/or system for analyzing molecules of an immunological nature, using optical biosensors based on the changes in interference due to the variation in the refractive index on the sensitive surface, in particular when working with real samples where the elimination of the matrix effect of the real sample, due to the high number of other existing agents that can generate a high non-specific adsorption, entails a very significant additional difficulty for competing with conventional chemical amplification and development systems such as those based on ELISA.

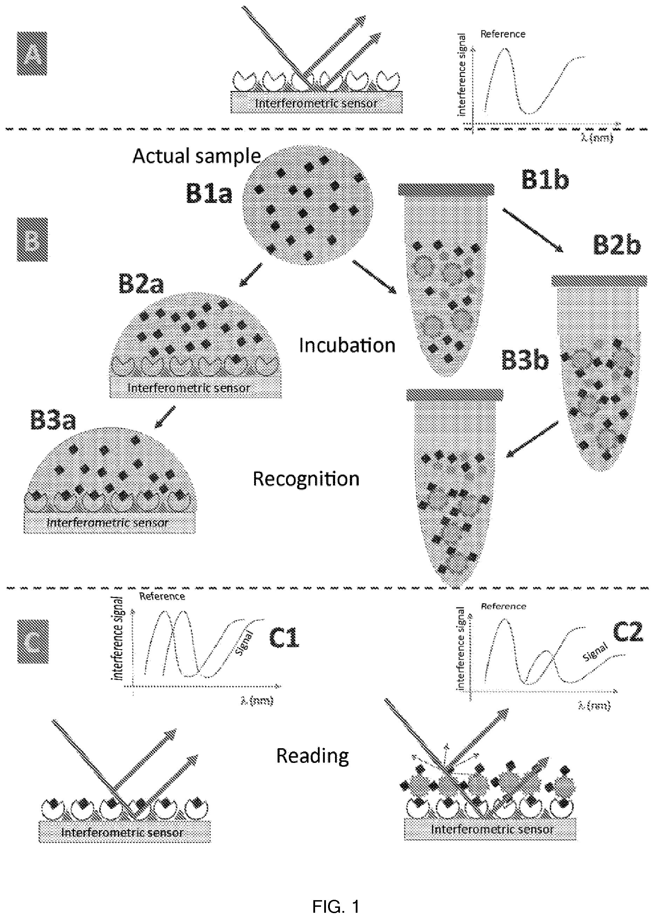

In this field of optical detection of biomolecules, in particular using the principle of optical interference, the transduction process is carried out on a functionalized surface that incorporates specific molecular receptors to exclusively recognize the target molecule, and blocking agents to prevent non-specific adsorption of other components found in the liquid biological sample *(*G. Zanchetta et al. "Emerging applications of label-free optical biosensors". Nanophotonics 2017; pp. 1-19*).* Said specific surface is part of the transducer which, once functionalized, increases its volume and/or density due to the presence of the specific molecular receptors and blocking agents mentioned, turning the sensor into a biosensor. This increase in the amount of matter generates a change in the interference response that we call the reference signal (see figure 1 A). When these molecular receptors recognize the target biomolecule, the amount of matter increases again (see figure 1C), in this case due to the increase in the specific biological material that this surface has been able to recognize. These molecules recognized in the transducer form a biological layer on the surface of the transducer or biosensor, with a thickness of a few nanometers depending on the type of target molecules.

A technical problem of great difficulty is the specific recognition or detection of molecules in a real sample, especially when these target molecules have a small size. Likewise, the processes of receptor functionalization (biofunctionalization) are also highly difficult. However, despite the difficulties, a high number of examples for the anchoring of specific bioreceptors are reported in the literature, for example by direct adsorption using polymer base materials by direct anchoring of antibodies and peptides, or by using reactions such as streptavidin and biotin, or by using protein A and protein G to orient antibodies in the biofunctionalization process. Other alternatives for making covalent surface anchors may be based on silanization processes, avidin-biotin reactions, among others that may be applicable to oxides, nitride, silicon, etc. Multiple biofunctionalization pathways can be found in Bioconjugate Techniques, Greg T. Hermanson, Second Edition, 2008, ISBN 978-0-12-370501-3*.*

In the case of the optical interference transducers object of this invention, the materials employed form a micro/nano photonic structure that generates an interference or resonance pattern. This interference or resonance pattern will be modified both in wavelength, frequency or wave number, as well as in intensity or amplitude of the signal by the anchoring and molecular recognition reactions, which makes the change the basis of the reading in the transduction of biochemical sensing (W. Holgado et al. "Description of an Advantageous Optical Label-Free Biosensing Interferometric Read-Out Method to Measure Biological Species. Sensors 2014, 14, 3675- 3689*).* The photonic structures most used for this type of sensor are those based on Match Zehnder interferometers, resonator rings and discs, Fabry-Perot interferometers, bimodal guides, nano-resonator networks or resonant nano-pillar networks, etc., such as those available in G. Zanchetta et al. "Emerging applications of label-free optical biosensors". Nanophotonics 2017; pages 1-19*.*

As mentioned above, the operating principle of an interference-based biosensor is the fact that when the bioreceptors are anchored to the surface of the interferometer, the interference signal changes, for example generating a change in the Interference signal caused by the sensor (figure 1A). When this biosensor is subjected to a recognition process, the bioreceptors specifically capture the target molecules causing said interference profile to be modified again, for example, a resonance or an interference minimum may shift and change its position in wavelength (see figure 1C). One way of measuring sensitivity may be by evaluating the wavelength shift of an interference minimum based on the number of target molecules specifically detected or recognized on the sensor surface. These relative variations of the resonance mode with respect to its initial reference (see figure 1C) determine the target molecules recognized on the surface, and therefore, allow to easily establish the concentration quantitatively in an analyzed sample from the realization of a calibration curve with known samples for which the concentration of origin is known. This method of proceeding to obtain quantitative information is widely reported, for example in R.L. Espinosa et al., "A Proof-of-Concept of Label-Free Biosensing System for Food Allergy Diagnostics in Biophotonic Sensing Cells: Performance Comparison with ImmunoCAP. Sensors 2018, 18, 2686*.*

However, these changes in wavelength, frequency or wavenumber related to the concentration of the biomolecule or molecule to be detected are limited by the response of the photon structure used to manufacture the sensor producing the optical interference. In addition, the dynamic range of concentrations that the sensor can measure depends on the construction thereof, where the detection area of the sensor plays a fundamental role. It is an unquestionable fact that non-specific adsorption on the surface of the sensor of the multiple and diverse components found in a real sample represents a very relevant difficulty that sometimes requires the use of blocking agents, and is a fundamental aspect when working with real biological samples of various nature, such as: blood, serum, saliva, tears, urine, among others, given the high number of different biological components or agents that are found together with the specific molecule to be specifically detected. For this reason, when developing a biosensor specificity is one of the most critical aspects, the elimination of this specific adsorption or the aforementioned matrix effect being a key factor to allow working with real samples. In this sense, a good and efficient functionalization or upholstery of the surface of the sensor can reduce the aforementioned matrix effect, although in many cases it is necessary to use blocking agents of various types such as serum albumin, ethanolamine, among others.

The invention described herein resolves the limitations of interference-based optical biosensors known to date in terms of their limited sensitivity, insufficient detection limit, lack of specificity, and difficulty in working with real samples due to the matrix effect. Thus, an optical method is described herein of detecting a target molecule by amplifying the interference response by refractive index and variation of the light intensity of this response as a result of the change of the refractive index itself and by the possible scattering produced by the type and size of the conjugates based on the NPs used for the performance of the assay. This method can be used for the measurement of target molecules of very low molecular mass, allows working with real samples, that is, samples that have not been subjected to a pre-treatment, as the specificity is increased and, above all, allows modifying the dynamic range of response and the sensitivity to demand of the bio-amplification to be used.

### DESCRIPTION OF THE INVENTION

The present invention provides an optical method for the detection of at least one target molecule based on biological interactions of said molecule. In particular, the method is based on the amplification in the interference response due to the variation in the refractive index generated by the NP conjugates used in the biological recognition process added to the possible further amplification caused by the phenomenon of scattering of said NP conjugates whose number on the sensor surface depends on the biological interactions of the target molecule in the different stages of the method. Accordingly, the method described herein allows qualitative and/or quantitative analysis of a target molecule in a real sample, in particular in a complex-matrix biological sample, improving the limit of detection by signal amplification caused by the variation of the interference signal and the specificity of the interference-based optical biosensors due to the possibility of separation of the NPs conjugates from the real sample to avoid the multiple components of the biological sample at their origin.

The method of the present invention makes it possible to improve optical interference sensing compared to the usual methods reported in the literature for interference-based optical sensors or transducers, which herein is also referred to as an interferometric sensor or transducer. In turn, this method could be used totally or partially in most of the optical biosensors based on interference or optical resonance reported in the literature, such as Fabry-Perot interferometers, resonator rings and disks, Match Zehnder Interferometers, BICELLs or bimodal guides, among many others. Thus, these devices will be able to compete and improve the performance of classic detection systems by chemical amplification or development, generally based on fluorescence or colorimetry (eg. ELISA).

In particular, the present invention provides an optical method for the detection of at least one target molecule (TM, also referred to as analyte) in a sample, characterized in that the method comprises the following steps:
a) Measuring the interference response or reference signal of an optical sensor or interferometric transducer with its biofunctionalised sensor surface (figure 1A) with:
   I. the target molecule (TM),
   II. or at least one specific bioreceptor (BR or BR1) of the target molecule.
b) Putting in contact, in a liquid medium, a sample to be analyzed and functionalized nanoparticles with at least one specific bioreceptor (NP-BR) of the TM (Figure 1B1b), preferably at a temperature between 2 and 37°C, forming a conjugate (NP-BR-TM) with the functionalized nanoparticles and the target molecules present in the sample;
c) Separating the NP-BR conjugates from the sample and, provided that the sample comprises the target molecule, the NP-BR-TM conjugates, since both conjugates will be together in the mixture obtained after step b (figure 1B3b);
d) Contacting said NP-BR conjugates, and if applicable, the NP-BR-TM conjugates obtained in step b) with said biofunctionalized sensor surface of the interferometric transducer (figure 1C2);
e) Determining the optical reading, in particular the interference response by reflection and/or transmission, from the variation of said interference profile caused by the variation of the real part of the refractive index and/or the variation of intensity caused either by the scattering or variation in the complex part of the refractive index of the aforementioned NP conjugates, or a combination of the foregoing, on the sensor surface of the optical transducer.

The aforementioned NP-based conjugates that are recognized on the sensor surface of the transducer will cause a change in the refractive index, mainly in its real part, which causes a change in the interference of the reflected or transmitted light. However, it may also occur that changes are generated in its complex part (depending on the type of NPs used), thus generating a change in the extinction coefficient and a reduction in the intensity of the reflected or transmitted light. In addition to this effect the scattering of the cited complexes of NPs can cause scattering of the light to a greater or lesser degree depending on the type and size of the NPs, and therefore, another change of the intensity of the received light, both in transmission and in reflection, which are added to the aforementioned effects.

The method of the present invention allows the detection of any TM, including those of very low molecular mass difficult to detect for optical biosensors, having at least one specific bioreceptor. Thus, this method can be used to detect any target molecule that may result in an immunological reaction or present bioreceptor-antigen affinity. In particular, the TM may be an antibody, for example an allergy-specific antibody (IgE) to a specific allergen or any other protein, hormone, toxin, among others. As one of the practical examples of this invention, an antibody specific to an allergy molecule (IgE) has been detected as TM, in such a way that the NP-antilgE conjugate (NP-BR in its initial notation in the present invention) that would recognize the IgE present in the biological sample forming the NP-antilgE-IgE complex (NP-BR-TM in its initial notation in the present invention), and on the surface of the sensor at least one allergenic molecule (BR1) has been immobilized that is recognized by the specific IgE of the aforementioned antibody (BR1 in its initial notation in the present invention).

The optical detection method described herein can be used in any sector where specific detection of an *in-vitro* molecule is required. Therefore, application sectors of great relevance can be, in addition to the clinical, the agri-food sector, where the detection of diseases in animals is required, or the detection of pathogens in their food or in their environment. Additionally, it can also be applied in the analysis of water in aquaculture. Other applications of the method are, for example, for process and quality control of products such as milk, wine, oils, as well as the detection of contaminants in agriculture and water monitoring, among many others.

Thus, in the method described herein, the sample to be analyzed can be a biological sample such as, for example, blood, serum, urine or tears among others; an agro-food sample such as, that which is obtained directly from food, or from the environment where the animals are located; or a water sample, either for human, animal or any other use. As mentioned above, the method of the invention can be applied to a real sample, i.e. a sample taken directly, without any pre-treatment. Although the application of the method to a liquid sample is preferred, in those embodiments in which the sample to be analyzed is a solid sample, the sample will be subjected to a previous step of dissolution in a liquid medium.

The functionalized NPs used in the method of the present invention are upholstered, i.e., functionalized on their surface, with at least one TM-specific BR, in particular a BR capable of specifically interacting with the TM (or analyte) by an immunological reaction, an antibodyantigen affinity reaction or a BR-TM reaction in general. Thus, on those occasions when the sample, preferably an untreated biological sample, comprises the TM, in step b) of the method of the present invention an affinity reaction takes place between these MOs and the NP-BR conjugates, resulting in the formation of NP-BR-MO conjugates. This step of the method can be performed at a temperature preferably between 2 and 37°C. Within this range, higher temperatures favor the immune reaction between the BR and the TM, requiring a shorter reaction time or incubation. Therefore, this time may vary, depending on the temperature in the incubation process, between minutes and hours, depending on the association constant and the kinetics of the recognition reaction and affinity of the bioreceptor used. In general, an incubation time of 30 min is usually set, although depending on the biological application, temperature, and kinetics of the particular reaction, this time may typically range from less than 30 min to hours.

In step c) of the method described herein, the separation of the NP-BR and NP-BR-TM (also referred to, respectively, as NP-BR conjugates and NP-BR-TM conjugates herein) generated in the affinity reaction taking place in step a) takes place, provided that the sample analyzed comprises TMs. Both conjugates (NP-BRs and NP-BR-TMs) are present in the resulting mixture to a greater or lesser degree. For such separation, any physical separation mechanism of the aforementioned NPs conjugates from the liquid medium of the sample to be analyzed can be used, such as for example centrifugation, separation by electrical or magnetic field, among other possible mechanisms. In particular, these NP conjugates can be readily separated from the rest of the sample by filtration, by means of one or more centrifugation cycles.

In particular embodiments, the NPs used may be capable of being attracted or repelled in the presence of an electric field due to the surface charge. In this case, step b) of separating the NP-BR and NP-BR-TM conjugates from the rest of the sample can preferably be performed by electric field and electrophoresis.

In other particular embodiments, the NPs used may be attracted or repelled by a magnetic field. In this case step b) of separating the NP-BR and NP-BR-TM conjugates from the rest of the sample can preferably be performed by application of a magnetic field.

Additionally, the NPs used may exhibit the ability to be attracted or repelled in an electrical or magnetic field, e.g., silica NPs coated with a magnetic material. In these cases a mixed separation mechanism based on the application of electric field, magnetic field and, optionally, centrifugation can be used.

As mentioned above, in step d) of the optical method of detection (*in vitro*) of a target molecule in a sample, the NP-BRs and the NP-BR-TMs, if any, are put in contact because the recognition of TMs present in the sample has occurred, obtained in step c) with a sensor surface of a biofunctionalized optical transducer (upholstered with TM or BR1) whose response, in particular, an optical sensor whose response is based on interference, resonance that generates a certain photonic structure such as those cited in the bibliographic references mentioned herein. In particular, any optical sensor operating by optical interference whose response changes in the presence of the material recognized on its sensor surface (the aforementioned NP conjugates in the present invention) can be used. Likewise, this interference or resonance response as a function of wavelength, wave number or frequency, not only changes due to the variation of the real part of the refractive index or optical thickness (referred to the refractive index of the NP conjugates multiplied by the average thickness increase of these NPs on the sensitive surface of the transducer), but also its amplitude (usually measured in optical intensity) can change as a function of the variation of the complex component of the refractive index and/or of the scattering caused by the NP conjugates recognized, and therefore located, on the surface of said sensor.

In particular embodiments, the sensor employed is an interferometric sensor that operates by measuring the variation of the interference, which is monitored by the change of light intensity in a given spectral range. To do this, said transducer is tuned so that the measurement is due to loss of light intensity (W. Holgado, et al. Sensors and Actuators B 236 (2016) 765-772*).*

Additionally, the NPs and the biological material accumulated or recognized on its surface increase the refractive index of the transducer, causing this response to be modified and, therefore, the intensity to change. In addition, the NPs linked to the biological material, can generate scattering losses in such a way that this phenomenon can be added to the loss of intensity generated by the change in interference.

It should be mentioned here that in the optical response by interference of photonic sensors, the refractive index plays an important role in the diversity of types of sensors reported in the technical and scientific literature. When on the sensor surface of a photonic transducer a conjugate of NPs are recognized and, due to this recognition phenomenon, specifically adhere, the average refractive index of the transducer is increased. In the case of sensors based on interferometers based on thin sheets of the Fabry-Perot type, the refractive index product by the average thickness of NPs causes the interference response to change (e.g. New Device Based on Interferometric Optical Detection Method for Label-Free Screening of C- Reactive Protein, IEEE Transactions on Instrumentation and Measurement, 68, 9, 3193- 3199, 2019*),* and in the case of those based on evanescent field detection, usually of horizontal optical interrogation and based on waveguides, the surface concentration of NPs on the surface increases the average refractive index that the light sees when propagating in a certain waveguide. In both cases, as discussed, a change in the interference profile is generated, such as Label-free optical biosensing with slot-waveguides, Optics Letters, 33, 7, 2008*.* Moreover, the imaginary part of the refractive index generates signal losses that can be identified as a loss of amplitude observed by signal intensity reduction. Finally, and mainly, for vertical interrogation sensors, the scattering losses will also reduce the amplitude of the interference signal and, therefore, can be used as a detection mechanism.

Thus, the method described herein can be used primarily in vertical optical interrogation interferometers, such as those based on Fabry-Perot interferometers, sensitive cells based on nano-structured structures, sensitive cells based on nano-pillar networks, and resonant nano-pillars, among others (e.g. Label-free biosensing by means of periodic lattices of high aspect ratio SU-8 nano-pillars, Biosensors and Bioelectronics 25 (2010) 2553-2558*;* Bio- Photonic Sensing Cells over transparent substrates for anti-gestrinone antibodies biosensing, Biosensors and Bioelectronics 26 (2011) 4842- 4847*; or* Resonant nanopillars arrays for label-free biosensing, Optics Letter, 41, 23,2016*).* Likewise, optical interrogation in plane interferometers can be used, such as Match- Zehnder interferometers, resonator rings, bimodal guides, grid guides, resonator discs, etc. such as those described in the aforementioned reference G. Zanchetta et al. "Emerging applications of label-free optical biosensors". Nanophotonics Nanophotonics 2017; 6(4): 627-645. DOI 10.1515/nanoph-2016-0158*; or* M. C. Estevez, M. Alvarez, and L. M. Lechuga, Laser Photon. Rev. 6, 463 (2012*).* Due to its simplicity, the use of a Fabry-Perot vertical interrogation interferometer is preferred.

This step c) of the method can be performed at a temperature between 0 and 40°C, preferably at a temperature between 2 and 37°C, and even more preferably, between 18 and 37°C when working in continuous flow systems where there is no evaporation. However, when working with sample droplets and, depending on the volume, thw work can be performed at temperatures between 2 to 37 °C (incubation process) using incubators to avoid evaporation and to favor the immune reaction by reducing said incubation time between the conjugates and the sensor surface of the biosensor. On the other hand, the temperature can be reduced to alleviate the evaporation factor, avoiding the need to use incubators. Depending on the option chosen, the incubation time may range from several minutes to hours, depending on the association constant and the kinetics of the recognition reaction and affinity of the bioreceptor used

The sensor surface of the optical transducer or sensor as cited in the present invention may be functionalized to have, immobilized on the surface of the sensor, the TM or, alternatively, at least one BR1 that also recognizes the TM. Additionally, the surface of said sensor may or may not comprise one or more blocking agents (BA) on the voids that would not have been covered, either by the TM or by the BR1, in order to prevent a non-specific response of other components that may be present in the sample to be analyzed.

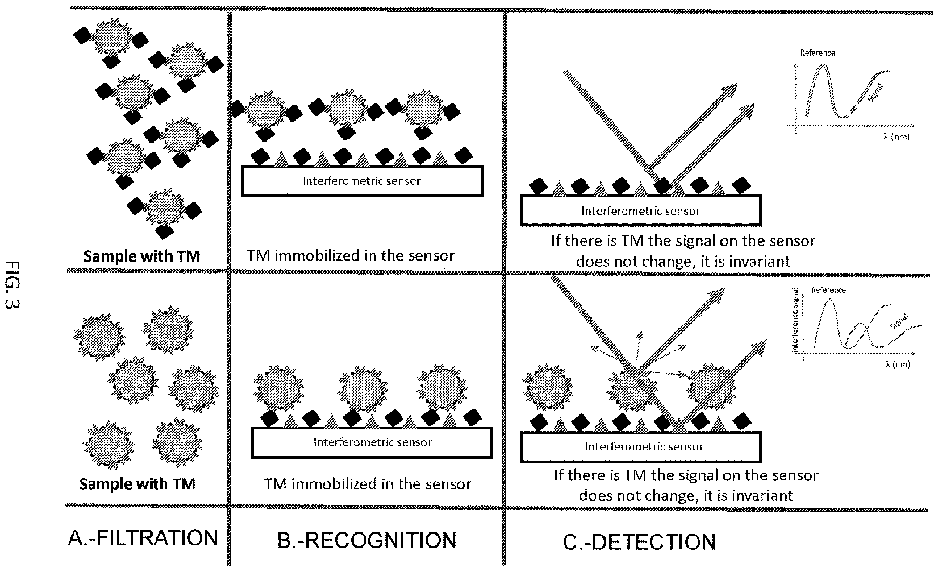

In those embodiments in which the sensor surface comprises the TM, i.e., this molecule is immobilized or upholstered on the sensor surface, the maximum variation of the optical reading signal is obtained by the mechanisms described in the present invention when the analyzed sample does not contain the TM, since in this case the number of NP-BRs that interact and are recognized on the sensor surface is greater (see diagram in the lower right part of figure 3). However, when the sample analyzed comprises TM, if this analyte is in a concentration sufficient to upholster all NP-BR conjugates, the variation of the signal will be practically zero, i.e. it will not be altered before and after step d). Ultimately, the response curve of the system will evolve with the signal decreasing as the concentration of TM in said sample increases. Therefore, in the event that the amount of TM in the sample is equal to or greater than the amount necessary to completely cover the surface of the total amount of NP-BRs used in the method described herein and, consequently, the filtrate obtained in step c) only comprises the NP-BR-TM conjugate, the optical reading signal will not be modified (see diagram in the left part of Figure 3). Quantification is carried out by calibrating the sensor between these two states. To this end a calibration curve is obtained in which the response signal of the sensor is obtained according to certain tabulated concentrations. It should be noted here that the ratio of NPs to TM present in the sample can now be easily modified to tune and adapt the dynamic range of the sensor.

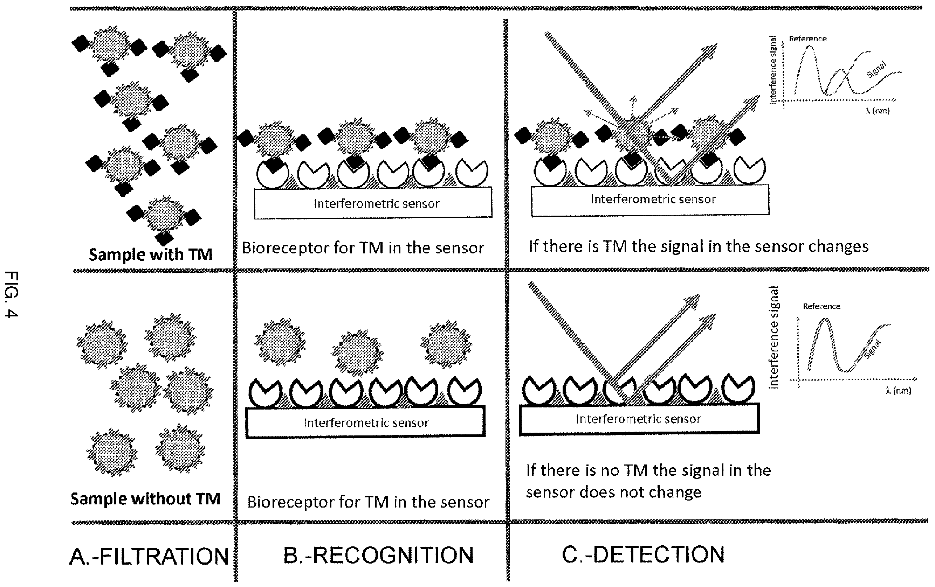

On the other hand, in those embodiments in which the sensor surface is functionalized or upholstered by the immobilization of at least one type of BR1 that specifically recognizes the TM, that is, a biomolecule (BR1) capable of specifically reacting with the TM, the change of the optical response of the sensor will increase as the concentration of TM in the analyzed sample increases (see upper left of figure 4), while said reading signal will not be modified when the sample does not contain the analyte or TM to be detected, since in this case the NP-BR-TM conjugate is not formed (see lower right of figure 4) and is not recognized by the surface upholstered by the aforementioned BR1. As in the previous case, TM quantification is performed by calibrating the sensor between the two situations that are exemplified in figure 4. To do this, a calibration curve is obtained in which the response signal of the sensor is obtained based on certain tabulated concentrations.

The specific bioreceptors (BR1) comprised on the sensor surface of the optical interference sensor may be the same or different from the bioreceptors (BR) comprised on the functionalised nanoparticles (NP-BR).

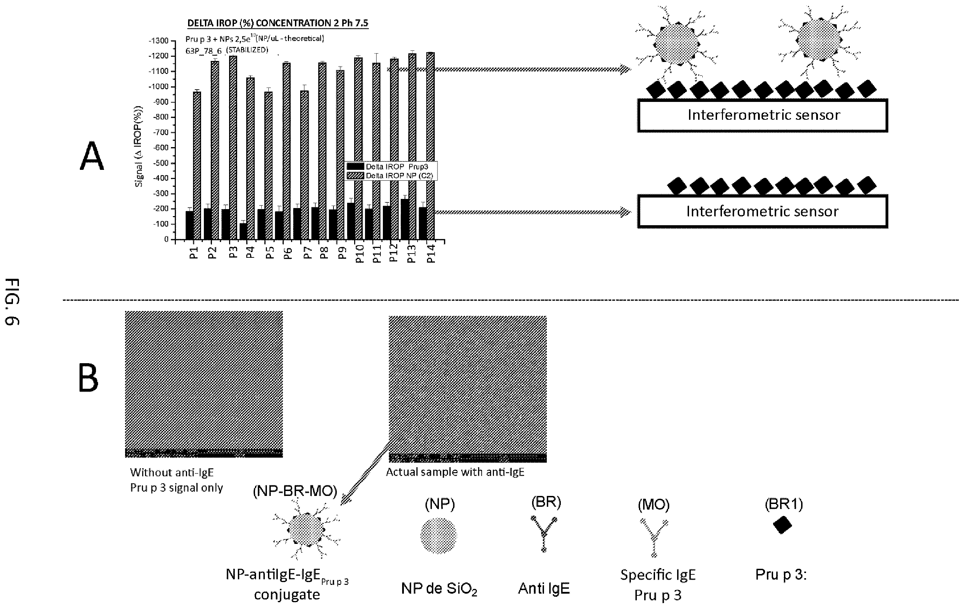

Additionally, in other particular embodiments of the invention, the disclosed method can be used to detect allergy specific antibodies (Ig), noted as TMs in the present invention, of a specific allergen in a biological sample. In these embodiments, the BR immobilized on the surface of the NPs (NP-BR) is a specific bloreceptor (anti-lgE) that recognizes the allergy-specific antibodies (i.e., the analyte or TM, in this case the IgE) in the biological sample to be analyzed. In this complex case, the NP-BR conjugate (NP-antilgE) captures the IgEs in the patient sample forming the NP-antilgE-IgE conjugate (noted as NP-BR-TM). In one embodiment of the present invention, when the specific receptor (BR1) is an allergenic molecule (AM), and in a particular case, Pru p 3 in the assay depicted in figure 6 herein, the sensor surface is upholstered with AM, such that if the NP-BR-TM conjugate (i.e., NP-antilgE-IgE) is recognized on the surface of the sensor, the signal will be zero when the patient does not have immobilized AM-specific IgEs on the sensor, while in the case that the patient has allergy-specific antibodies, the NP-BR-TM conjugate (i.e., NP-antilgE-IgE) is recognized on the surface of the sensor, altering the optical reading signal as described in this invention. Ultimately, in these embodiments, if the patient from whom the analyzed sample has been obtained has an allergy, the interference signal will be modified by the presence of the NP-BR-TM conjugate, where the TMs are the allergy-specific antibodies (g) to the allergenic molecule (AM). In this way, the method of the present invention is a great advantage, since it allows the *in vitro* detection of possible allergies that a patient may have. This determination is currently performed *in vivo* using the prick test, which consists of introducing allergenic molecules or extracts into the patient's arm and observing if the body reacts.

Thus, in the present method two mechanisms are involved. On the one hand, the change of the interference in wavelength, frequency or wave number of the interference profile when the optical path is increased (refractive index in its real part times the length that the light must pass through), and on the other hand the possible reduction of the amplitude by the scattering of the light of the corresponding NP complexes on the surface of the sensor due to the aforementioned mechanisms of scattering and/or variation of the complex part of the refractive index.

The method of the present invention allows quantitative analysis, i.e. determining the presence or not of a target molecule (TM), as well as its quantification depending on the assay to be performed on a sample.

The nanoparticles used in the method of the present invention may be formed of any type of dielectric inert material, such as oxides or silicon; any material transparent to light, for example, silica, titania, alumina or silicon; as well as nanoparticles of other nature such as gold, aluminum, silver, among others. In the latter case it occurs that at certain wavelengths the NPs can be transparent to light and/or generate more or less scattering or variation in the extinction coefficient or complex part of the refractive index. Thus, the nanoparticles may be selected from the group consisting of silica, gold, aluminum, silver, silicon, and metal oxides, in particular titanium oxide (titania) or aluminum oxide (alumina).

It should be noted that the size and concentration of NPs are calculated based on the surface of the sensor, the type of interference biosensor used and the dynamic range of the concentration to be detected.

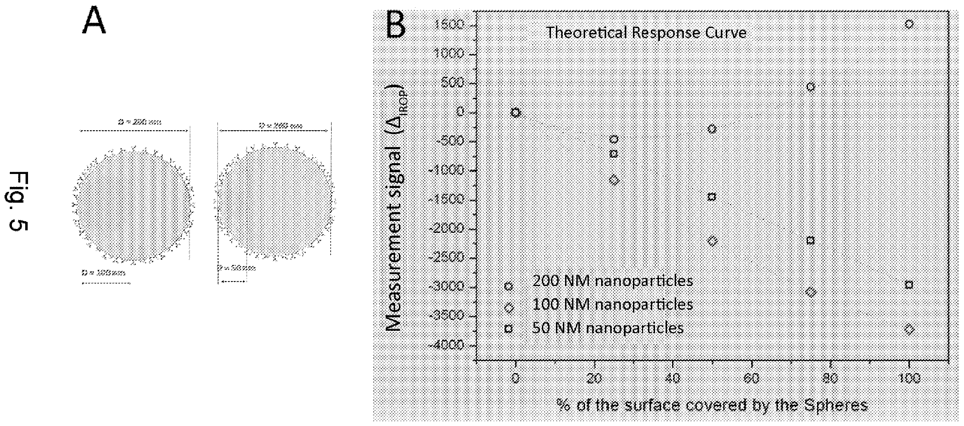

Therefore, in preferred embodiments of the method of the present invention, when the optical sensor used is a Fabry-Perot interferometer, the use of spherical silica NPs (SIO₂) with a diameter between 50 and 100 nm, or of another shape with diameters of the same magnitude as the previous ones, preferably with a concentration of 10⁸ to 10¹² (1 E8 to 1 E12) nanoparticles per micron is preferred. This concentration can vary, mainly depending on the sensitive area of the sensor, incubation time, temperature and volume, since it is not only the kinetics of the reaction that determines the association time, as the diffusion and sedimentation of the NPs also play an important role. The higher the concentration of NPs, the less time it takes for them to reach the surface and be able to upholster the sensitive area of the sensor. As shown in figure 5, when the NPs have a diameter of 200 nm, the response (□Iᵣₒₚ signal from the Fabry-Perot sensor used) is not monotonically decreasing and only with an upholstery equal to or greater than 50% can the sensor response be used. In this case, it is considered that the NPs are not tuned and the response of the biosensor system does not improve. However, when the NPs are spherical with a diameter of 50 nm or 100 nm, it is observed that the response signal is monotonically decreasing and the sensor signal (□ Iᵣₒₚ signal of the sensor used) decreases almost linearly with the area upholstered in the sensor. In this case, the biosensor system is tuned and the sensitivity has been improved because the slope of the response curve is higher than that reported in previous works, thanks to the method of the present invention.

In other preferred embodiments of the method of the present invention, when the optical sensor used is a Fabry-Perot interferometer, the use of gold (Au) NPs with a diameter of 20 to 70 nm and preferably 45-55 nm is preferred, sizes that make the NPs transparent to the wavelength or spectral range of optical interrogation *(*Modelling the optical response of gold nanoparticles, Chem. Soc. Rev., 2008, 37, 1792-1805*)* and, preferably, a concentration of 10⁸ or 10¹¹ NPs/mL. This concentration can vary, mainly depending on the sensitive area of the sensor, incubation time, temperature and volume, since it is not only the kinetics of the reaction that determines the association time, as the diffusion and sedimentation of the NPs also play an important role. The higher the concentration of NPs, the less time it takes for them to reach the surface and be able to upholster the sensitive area of the sensor.

The relevance of the Invention is that the modification of the refractive index is produced by the biological material immobilized or recognized in the NP, since it acts as a vehicle and it is said biological material that changes the refractive index of the transducer, and therefore, its interference response. This interference response is read by the variation of intensity of a wavelength or a spectral range and, depending on the size of the NP with its corresponding biological material, the effect of scattering is added

The method of the present invention allows great versatility, since it is possible to select the size and material of the nanoparticles (NPs) that allows tuning with the interference response of the transducer. Through this tuning process, the interference response can be amplified in the chosen transducer, preferably a Fabry-Perot interferometer, as well as taking into account the reduction of the signal amplitude due to the scattering of the NPs that are recognized in the sensor. Additionally, the method of the present invention also makes it possible to apply design criteria to determine the amplification constant, establish the quantitative detection range, significantly improve sensitivity, reduce measurement uncertainty, and drastically improve the detection limit. Additionally, this method also makes it possible to modify the reading system of the detection signal, which makes it possible to reduce the uncertainty of the measurement.

Thus, the following advantages are achieved with the detection method described in this patent application:
- Detection of molecules of both low and high molecular mass, since the method is independent of the molecular mass of the target molecule (TM) to be detected and an unresolved technical solution to a common problem is given. More specifically, in the optical method described herein detection depends on the conjugates (NP-BR or NP-BR-TM) recognized on the sensor surface. Thus, unlike conventional methods where the mass on the sensor surface is modified very little, if the TM is of very low molecular mass, in the method of the invention the mass on the sensor surface depends mainly on the nanoparticle (NP) and the biorreceptor (BR), and not so much on the molecular mass of the target molecule (TM). This is a major advantage over other methods using interference sensors, where the signal and sensitivity are highly dependent on the molecular mass of the target molecule.
   - It allows working with real samples, i.e. samples (in particular biological samples) that have not undergone any pre-treatment. The matrix effect problem, non-specific adsorption, is drastically reduced or eliminated, since the method comprises separating the analyte from the rest of the sample and, advantageously, this separation is done outside the sensor, so that only a clean sample is deposited on the sensor surface of the biosensor. This eliminates the need to use complex locking systems on the surface of the sensor itself, in addition to avoiding complex washing processes directly on the sensor to eliminate the matrix effect or non-specific adsorption. It should be noted here that the process of blocking and labelling in optical systems that work without optical labelling is perhaps one of the main reasons why they have not been massively marketed as a sensor or diagnostic system in the point of care.
- By selecting the quantity, size and material of the NPs it is possible to optimize the response curve, represented as the measurement signal with respect to concentration, in order to adapt the response to the range of concentrations to be detected, that is, the dynamic range. In this way, it is possible to optimize the range of concentrations in which it is desired to work based on the selected biological application and for each type of transducer selected that operates by means of interference
- This method dramatically improves the sensitivity and detection limit of the interference transducer being used. Thus, the method allows detecting minimum concentrations of the target molecule (analyte) due to the increased sensitivity in the transduction process. Additionally, by selecting the amount, size and material of the NPs it is also possible to significantly improve the detection limit, since the amplification of the signal depends on the NPs used, while the reading uncertainty of the system does not depend on the NPs. Consequently, the Sensitivity/Uncertainty ratio corresponding to the detection limit can be improved for any reading system due to said increased sensitivity.

In conclusion, the present invention solves important problems of optical biosensors operating by interference:
I. With this invention the sensitivity of the detection system no longer depends on the molecular mass of the target molecule to be detected or recognized.
II. The sensitivity is drastically increased, thus improving the limit of detection in a very significant way to detect molecules of very low molecular mass in small concentrations
III. It allows effective measurement of real samples (e.g. complex matrix biological samples) by reducing or eliminating the non-specific signal (also called background signal) and thus be able to make measurements
IV. Moreover, the response curve can be adapted to the range of concentrations to be measured.

Thus, the method of the present invention can be used to develop *in-situ* and non-invasive diagnostic systems by capturing biomarkers present in different, preferably biological, samples. In the case of clinical diagnosis, the biological liquid samples where to find these biomarkers may be, among others, blood, serum, urine, tears among others as medullary cerebral fluid, water, saliva, etc. Likewise, the diagnostic method may be used for multiple sectors where a detection of bioreceptor-analyte recognition takes place *in-vitro* (e.g. antibodyantigen affinity reaction).

Thus, the invention also provides an immunological-based *in-vitro* diagnostic method using optical interference-based photonic transducers (interferometric transducers), wherein these transducers comprise on their sensor surface: i) the target molecule, or ii) at least one specific bioreceptor (BR1) thereof; as well as a reading system capable of monitoring the relative variations of the immobilization and/or recognition events that are drastically amplified by the use of the NP-BR and NP-BR-TM conjugates described above.

In particular embodiments of the present invention, the sample to be analyzed is a biological sample, either human or animal, and the target molecule to be detected is a biomarker that allows *in vitro* diagnosis of, for example, a disease.

In one of the preferred embodiments, the optical detection method of the present invention may be used for *in vitro* diagnosis of food allergy. This method would consist in the detection of allergy-specific immunoglobulins (IgE) contained in real samples of patients. In this case, the target molecules (TMs) to be detected are IgEs specific to the allergens to which the patient is allergic. To do this, several allergenic food molecules are immobilized in different wells of a biokit in which, for example, several Fabry-Perot type interferometers are found (e.g. Towards reliable optical label-free point-of-care (PoC) biosensing devices, Sensors and Actuators B 236 (2016) 765-772*).* NPs are biofunctionalized with a bioreceptor (BR) consisting of an IgG Inmonuglobulin that specifically recognizes all existing IgEs in the patient sample (sera from food allergy patients). In order to distinguish which IgE molecules the patient is allergic to, after the incubation and filtering of the patient's sample a sample is obtained that will contain the NPs conjugates with the different IgEs. This sample is deposited and incubated in the different wells containing the allergenic molecules for which the diagnosis is made, so that the NPs conjugates with the specific IgE bind to the corresponding allergen, which are detected when reading the optical signal by the change in the interference that is generated.

The detection method described herein, therefore, has to further comprise a prior step of functionalizing nanoparticles with at least one specific bioreceptor, resulting in the formation of the functionalized nanoparticles (NP-BR). Any anchorage method (covalent bond, direct adsorption or any other biofunctional pathway) known to the skilled person can be used to carry out this functionalization.

In those embodiments in which the NPs are silica, the preferred method of functionalization is silanization, to couple an anchor molecule that orients the antibody in its non-specific part. On the other hand, when the method uses gold NPs, it is preferable to use the thiol as an anchoring element. The mechanisms of surface biofunctionalization are extensively reported in the scientific literature (e.g. Bioconjugate Techniques, Greg T. Hermanson, Second Edition, 2008, ISBN 978-0-12-370501-3*).*

As a particular example, biofunctionalisation of silica NPs with specific bloreceptors of the target molecule can be performed from a monodispersed solution of nanospheres with amine groups on their surface. The carboxyl groups present in the antibodies can be activated and stabilized by the addition of EDC (1-ethyl-3- (3-dimethyl aminopropyl) carbomidine hydrochloride) and NHS (N- hldroxysuccinimide), which causes a spontaneous reaction with the primary amines present on the surface of the nanospheres, resulting in the formation of a stable NP-BR conjugate by the formation of a covalent amide.

Likewise, the optical detection method of the present invention also requires the biofunctionalization of the surface of the optical sensor, as described in step d) of recognition, in which the conjugates NP-BR-TM and/or NP-BR are put in contact with the sensor surface. Different routes have also been widely described in the literature for the biofunctionalization of the sensor surface of the transducers, with covalent or adsorption anchors, with or without orientation and using different blocking agents. Throughout this application several references have been cited in which these biofunctionalization routes that are not the object of the present Invention are described.

### BRIEF DESCRIPTION OF THE FIGURES

Figures 1A-1C: Figure 1A illustrates an optical signal from an interferometric transducer (e.g. Fabry-Perot type with Vertical Optical Interrogation) in which specific bioreceptors have been immobilized. Figures B1a-B3a are a schematic representation of the traditional operation process, while figures B1b-B3b refer to the process described in the present invention in which NPs conjugates are used. Figure C1 shows schematically the detection of a target molecule in an interference sensor, where the profile of the spectral response is modified by the recognition of a target molecule, while figure C2 shows how the detection would be when using what is described in the present invention, where the change of the spectral optical signal is significantly amplified, and also can lead to a modification in the aforementioned amplitude of the said optical signal, so that both effects significantly improve the detection capacity of the system.

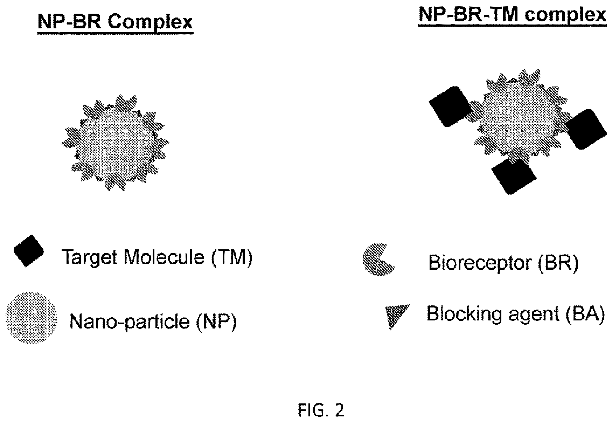

Figure 2: It is a schematic representation of the different types of conjugates formed with the nanoparticles (NP) in the method of the present invention, i.e., functionalized nanoparticles (NP-BR) and conjugate (NP-BR-TM) formed by these functionalized nanoparticles and the target molecule. In the particular embodiments depicted in this figure, both conjugates comprise a blocking agent (BA), however, the method of the present invention could also be selectively performed when the NP-BR and NP-BR-TM do not comprise any blocking agent.
Figure 3: This figure shows the detection mechanism when the TM is immobilized on the sensor surface. More specifically, in the left part of figure 3A the result of having incubated the NP-BRs (step b) with the sample and filtered (step c) to obtain the NP-BRs conjugates in the case that the biological sample did not contain the TM and NP-BR-TMs in the case that if there were, the extreme case in which all the NP-BRs present in the reaction medium capture all the TM present in the sample is exemplified here. Figure 3B depicts the recognition step (step d and step e), in the event that the sample did not contain TMs, NP-BRs conjugates would be recognized on the surface of the sensor and the signal would be maximum (see bottom of Figure 6C), i.e. the sensor interference signal changes significantly. In the event that the sample did have the TM, then the NP-BR-TM conjugates would not be recognized on the surface of the sensor since it also has the TM immobilized (see the top of Figure 6C)
Figure 4: This figure first depicts the recognition and filtering mechanism (figure 4), then the detection mechanism when what is immobilized is a specific receptor (BR) of the target molecule (TM) in the sensor (figure 4B). Contrary to what was described in figure 3, the sample containing the NP-BR-TMs conjugates will now be recognized on the surface of the sensor and therefore in this case the change of the signal by interference will be very significant (see upper part of figure 4C), while when the sample does not have the TM the NP-BRs conjugates will not be recognized on the surface of the sensor (see lower part of figure 4C) and therefore the interference signal will practically not be altered.
Figure 5: Example of tuning of NPs on a biosensor system. In this case, a Fabry-Perot type interference sensor has been used as a reading mechanism based on Increased Relative Optical Power (IROP (%)) described in Towards reliable optical label-free point-of-care (PoC) biosensing devices, Sensors and Actuators B 236 (2016) 765-772*.* The simulation of the response curve represents the reading signal as a function of the percentage of NPs recognized on the surface. It can be seen that for NPs from 50 to 100 nm, the response curve is monotonically decreasing and valid to be used as a sensor, while for 200 nm the response curve would not be valid.
Figure 6A: In Figure 6A the change of the signal in a real sample of serum with high concentration of specific IgE to Pru p 3 in 14 wells (P1 to P14) can be seen. Each well contains a Fabry-Perot type sensor. In Figure 6B, scanning electron microscopy (SEM) images can be seen, where an image is shown without NPs on the surface, and another with NPs on the surface. This is the case where the signal increase is observed and therefore the recognition of the NP-IgG-IgE-Pru p 3 conjugate is certified. It should be noted that in this figure the readings of the signals of a 14-well diagnostic kit (P1 to P14) in serum matrix (in this case expressed in □Iᵣₒₚ %) obtained in example 1 (see below) are shown. The lowest signal of these readings relates to the immobilization of the bioreceptor (BR1), while the high ones relate to the specific detection of the target molecule (TM), in this case a specific IgE of the allergen Pru p 3. As can be seen in this graph, the silica NPs used have been functionalized with a bioreceptor (BR) consisting of limunoglobulins G (IgGs) that specifically recognize all the immunoglobulins E (IgEs) present in the biological sample, i.e. the NPs have been functionalized with antilgE (IgGs that capture IgEs). The sensor surface is functionalized with a bioreceptor (BR1). In this case they are molecules of a specific type of allergen (Pru p 3), in such a way that only those IgEs specific to Pru p 3 (in this case the target molecule) will be recognized on the surface of the sensor, and therefore when this occurs the optical reading signal changes.

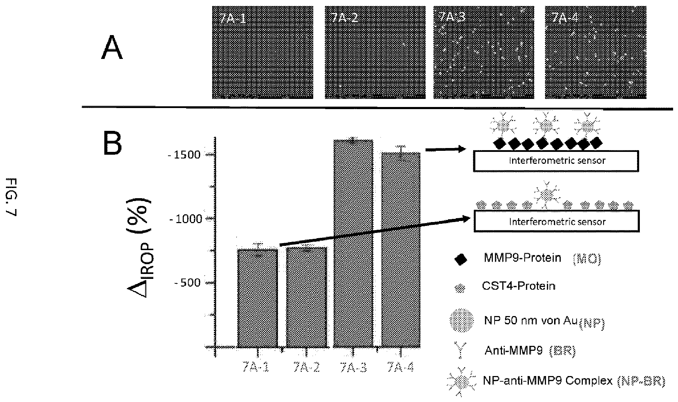

Figure 7A: SEM images of the conjugates recognized on the sensor surface (images 7A-3 and 7A-4) in contrast to images 7A-1 and 7A-2 where a limited number of NPs have been recognized. In this case, the method has consisted of using gold NPs that have been biofunctionalized with a bioreceptor (BR) consisting of an immunoglobulin G that specifically recognizes the Target Molecule (TM) that is the metalloproteinase MMP9. In this case the nonbiological sample did not contain the MMP9 and therefore the NP-antiMMP9 conjugates are recognized in the sensor, significantly increasing their signal (figure 7B); in addition it is verified that the high signal is due to the NPs that are observed by microscopy (images 7A-3 and 7A-4 of figure 7A). As an additional test, another TM (cystatln CST4) was immobilized on another sensor. When the sample was contacted with this other sensor, it could be observed that there was practically no recognition of the NP-antiMMP9 conjugates with the CST4, as can be seen by the limited number of NPs observed by microscopy (images 7A-1 and 7A-2 of figure 7A) and the smallest measured signal (figure 7B).

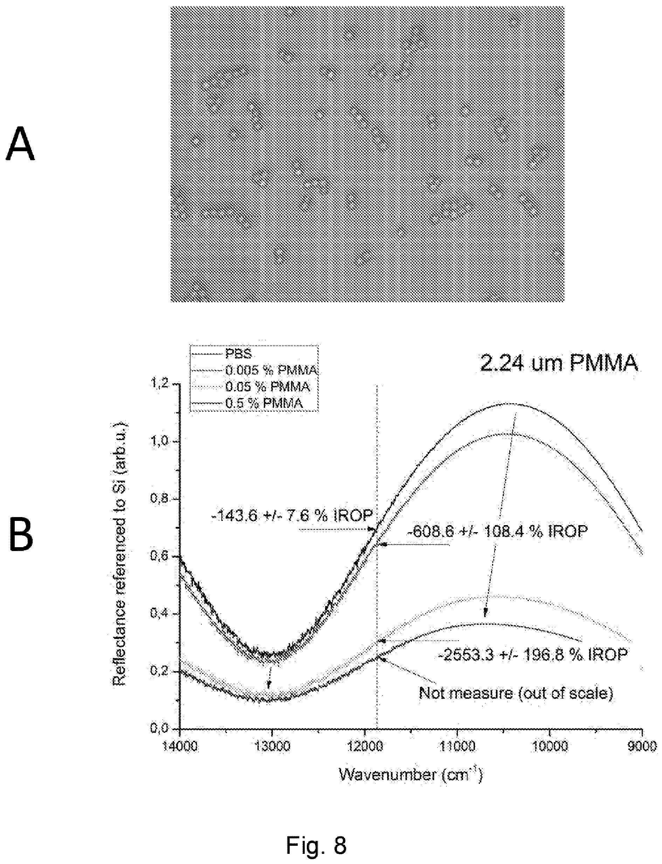

Figure 8: In this figure PMMA NPs are observed on the surface of a sensor by means of which an experimental test is carried out to certify that, depending on the size of the NPs, the scattering of light is also a phenomenon that can be used and/or added to the change of interference. In this case, by means of PMMA NPs, it can be observed how the interference profile is reduced in signal amplitude and used as a detection mechanism.

### EXAMPLES

In order to contribute to a better understanding of the invention, and in accordance with a practical embodiment thereof, several preferred embodiments of the present invention are accompanied forming an integral part of this disclosure.

### Example 1: Detection of food allergy specific IgE using silica nanoparticles

In this case, the NPs are functionalized by immobilizing a specific receptor of allergy specific antibodies IgE (NP-antilgE), it is advisable to indicate that the anti-lgE are IgGs that recognize and capture all the existing IgEs in the corresponding biological sample, and on the surface of the sensor one of the specific allergenic molecules is immobilized for which it is desired to know if the specific allergy antibody that is related to said molecule, and therefore detect if the patient has allergy specific antibodies (IgEs) to the Pru p 3 molecule. Measurements are made on a sample of real serum. In this case, the NP-antiigE-IgE conjugate is formed, but with the peculiarity that the IgE may or may not be specific to the allergenic molecule that has been upholstered in the sensor (Pru p 3, in this test) and that we have noticed as BR1. Thus, if the sensor signal increases, it means that the conjugate is recognized on the surface and the sample comes from a patient who has a specific concentration of allergy-specific antibodies to the Pru p 3 molecule. On the contrary, if the sensor signal does not change, the sample does not contain specific antibodies for allergy to Pru p 3.

An allergenic molecule (Pru p3) was immobilized on the sensor surface of the Fabry-Perot Interferometer Sensor at a concentration of 5 µg/mL.

On the other hand, an antibody (anti-lgE) was immobilized that specifically recognizes all IgEs in silica NPs (NP-antilgE conjugate) with a diameter between 50 nm and 100 nm.

A sample was taken from the patient, which was centrifuged to obtain the serum. Subsequently, this sample was mixed with the patient's serum, where the allergy specific antibodies (IgEs) were found. The functionalized silica NPs were added at a concentration of 2.5 × 10¹⁰ NPs/□L and incubated for 30 min, i.e. left at 37°C in an incubator for the NP-anti-IgE conjugate to recognize the possible IgEs present in the patient sample.

Once the incubation process was completed, the centrifugation process was carried out so that the NPs conjugates were sedimented in the Eppendorf flask. The supernatant was removed, thus obtaining the NPs conjugates that have recognized the patient's IgEs. This centrifugation process was repeated 3 times.

Once the sample containing the NP-antilgE-IgE conjugates (specific) was filtered, it was introduced into the sensor previously functionalized with the allergic molecule Pru p 3 and incubated for 30 min at 37°C. After the recognition step, if the NP-antilgE-IgE conjugate is specific to the allergenic molecule, the conjugate is specifically recognized on the surface of the sensor and the reading signal changes.

Figures 6A and 6B show the results obtained in this assay. In particular, in figure 6A it is possible to observe the immobilization bars of the Pru p 3 allergen and the increase of signal in 14 wells (signals of 1100 of □ Iᵣₒₚ (%) when the NP-antilgE-IgE conjugates specific to Pru p 3 of the biological sample is recognized on the surface of the sensor. In this case, signals of 1200 pp □ Iᵣₒₚ (%) are obtained, in contrast to the initial signal corresponding to the immobilization of the PRU p 3, signals of 200 □ Iᵣₒₚ (%)) This signal is corroborated with figure 6B, where scanning electron microscopy (SEM) images are shown that demonstrate the recognition of the NP-antilgE-IgE Pru p 3 conjugate.

It is important to note here that this sensor works by the intensity of the light generated by the Fabry-Perot interferometer used *(*Towards reliable optical label-free point-of-care (PoC) biosensing devices, Sensors and Actuators B 236 (2016) 765-772*)* and, in this case, the method described here amplifies the recognition signal, since it depends both on the displacement of the signal by interference, amplified by the Increase of matter in the sensor, as well as by the loss of intensity of the light by the optical scattering of the NPs recognized on said surface.

### Example 2: Detection of metalloproteinase MMP9 (inflammation marker)

To perform this experimental test, the MMP9-specific antibody (antiMMP9) was immobilized on the surface of the gold NPs (NP-antiMMP9 conjugate). In this case, 50 nm gold NPs were chosen since they are transparent to the range of wavelengths chosen around 850 nm *(*Modelling the optical response of gold nanoparticles, Chem. Soc. Rev., 2008, 37, 1792-1805*).*

On the other hand, Fabry-Perot interferometer type sensors were upholstered with MMP9 and CST4 on their corresponding sensor surfaces with a concentration sufficient to have a high percentage of upholstery of said sensor surfaces. Specifically, both the MMP9 protein (inflammation marker) and the CST4 protein were immobilized at a concentration of 10 □g mL⁻¹ on the surface of the Fabry Perot type sensor. Therefore, two biosensors are obtained, one in which a protein related to the antiMMP9 (the target molecule) has been immobilized and in another a protein not related to the MMP9 antibody.

In this case, to manufacture the interferometer, a pulmérlca base material (SU8) similar to that described in Development towards Compact Nitrocellulose-Based Interferometric Biochips for Dry Eye MMP9 Label-Free In-Situ Diagnosis Sensors 2017, 17, 1158; DOL: 10.3390/s17051158) was used, except that this case did not use nitrocellulose for anchoring.

The analysis was performed directly using NP-antiMMP9 conjugates that were meant to recognize MMP9 as bioreceptor and not to recognize CST4 as receptor. To do this, the sample containing the NP-antiMMP9 conjugates was incubated on the aforementioned sensor surfaces for a period of 2 hours, at a temperature of 37°C. So if the signal is high, the NP-antiMMP9 conjugates have been recognized on the surface, while if the signal is low, it is that the NP-antiMMP9 conjugates are not recognized on the sensor surface and the signal must be low.

Figure 7 shows the results obtained in this test. It can be seen in the electron microscopy images (see figure 7A) how the NP-anti-MMP9 conjugate is recognized on the surface (images 7A-3 and 7A-4, and therefore the target molecule (TM) is recognized. In addition, this is corroborated by the signals detected on the sensors in figure 7B (signals 7A-3 and 7A-4) that are very high when the aforementioned NP-antiMMP9 conjugates have been recognized. However, the results found when immobilizing a protein that is not related (in this case the aforementioned CST4) indicate that the number of NP-antiMMP9 conjugates is limited, and therefore, the signal is much lower. See figures 7A, images 7A-1 and 7A-2 and figure 7B, signals 7A-1 and 7A-2 respectively.

## Claims

1. An optical method for the detection of at least one target molecule in a sample, the method comprising:
a. Measuring the interference response of an optical sensor or interferometric transducer with its biofunctionalized sensor surface (figure 1A) with:
i. the target molecule (TM) to be analyzed comes from a sample selected from the group consisting of a biological sample, a clinical sample, an agri-food sample and water
ii. Or at least one specific bioreceptor (BR or BR1) of the target molecule;
b. Putting in contact, in a liquid medium, a sample to be analyzed with functionalized nanoparticles (NPs) with at least one specific bioreceptor (NP-BR) of the target molecule (TM), forming a conjugate (NP-BR-TM) with the functionalized nanoparticles and the target molecules present in the sample;
c. Separating NP-BR conjugates from the sample and, provided the sample comprises the target molecule, NP-BR-TM conjugates formed in the mixture obtained after step b);
d. Putting in contact said NP-BR, and if applicable, the NP-BR-TM conjugates obtained in step b) with said biofunctionalized sensor surface of the interferometric transducer; and
e. Determining the optical reading, measuring the variation of the actual part of the refractive index and/or the variation of intensity caused by the scattering or variation in the complex part of the refractive index of the NP-BR conjugates, and if applicable, the NP-BR-TM conjugates, or a combination of the foregoing, on the sensor surface of the interferometric transducer.

2. The optical detection method according to claim 1, wherein the target molecule is an IgE allergy specific antibody specific to an allergenic molecule (AM).

3. The optical detection method according to claim 2, wherein the BR1 is the allergenic molecule (AM) specific for an allergy specific antibody and the sensor surface is functionalized with said allergenic molecule (AM).

4. The optical detection method according to any one of claims 1 to 3, wherein the clinical sample to be analyzed is selected from the group consisting of blood, serum, plasma, saliva, tears and urine.

5. The optical detection method according to any one of claims 1 to 4, wherein the sample to be analyzed is a clinical sample and the target molecule is a biomarker for in vitro diagnosis.

6. The optical detection method of claim 5, wherein the biomarker is selected from the group consisting of proteins, hormones, immunoglobulins, toxins, or any molecule that is recognized by immunological processes.

7. The optical detection method according to any one of claims 1 to 6, wherein steps a) and c) take place at a temperature between 0 and 40°C, the temperature conditions of these steps being equal or different from each other.

8. The optical detection method according to any one of claims 1 to 7, wherein step b) of separation takes place by a technique selected from the group consisting of centrifuging, electric field separation, magnetic field separation, and a combination of the foregoing.

9. The optical detection method according to any one of claims 1 to 8, wherein the nanoparticles are selected from the group consisting of silica, alumina, silicon nitride, silicon, dielectric materials, metal oxides, magnetic materials, gold, aluminum, silver, and metallic materials.

10. The optical detection method according to any one of claims 1 to 9, wherein the optical sensor is a Fabry-Perot interferometer, and the functionalized nanoparticles (NP-BR) comprise spherical silica nanoparticles with a diameter between 50 nm and 100 nm.

11. The optical detection method according to claim 10, wherein the concentration of NPs is between 10⁸ to 10¹² NPs/µL.

12. The optical detection method according to any one of claims 1 to 9, wherein the optical sensor is a Fabry-Perot interferometer, and the functionalized nanoparticles (NP-BR) comprise gold nanoparticles with a diameter of between 20-70 nm.

13. The optical detection method according to claim 12, wherein the concentration of NPs is between 10⁸ to 10¹¹ NPs/mL.
